# EUROPEAN PATENT SPECIFICATION

(11) **EP 1 289 581 B1**
(45) Date of publication and mention of the grant of the patent: **19.07.2006**
(21) Application number: 01928182.3
(22) Date of filing: 03.05.2001
(51) Int. Cl.: A61M 5/30, A61N 1/30

(54) **ELECTRICALLY-MEDIATED TRANSDERMAL DRUG INJECTION**
ELEKTRISCH VERMITTELTE TRANSDERMALE MEDIKAMENTENINJEKTION
INJECTION DE MEDICAMENT TRANSDERMIQUE A ASSISTANCE ELECTRIQUE

(30) Priority: 07.05.2000 IL 13600800
(43) Date of publication of application: 12.03.2003
(73) Proprietor: Transpharma Ltd., 56217 Yehud (IL)
(72) Inventor: GROSS, Yosef, 73160 Moshav Mazor (IL); AVRAHAMI, Zohar, 76241 Rehovot (IL)
(74) Representative: Rottmann, Maximilian
(86) International application number: PCT/IL2001/000397
(87) International publication number: WO 2001/085234

(56) References cited:
- WO-A-95/15783
- US-A- 5 860 957
- US-A- 5 983 131

## Description

### FIELD OF THE INVENTION

The present invention relates to an apparatus for injection of a substance through the stratum corneum of skin such as defined in claim 1.

### BACKGROUND OF THE INVENTION

Methods for delivering a drug through a patient's skin are well known in the art, and include passive diffusion of the drug from a skin patch to the skin, and active processes such as hypodermic injection, iontophoresis, sonophoresis, electroporation, laser ablation, and chemically-enhanced diffusion. Each of these methods is typically limited by one or more of the following:
- a needle and/or tissue-heating causes the patient pain,
- tissue-heating causes unnecessary damage,
- generation of a hole in the skin and transfer of the drug are performed in two separate steps,
- expensive apparatus is required,
- only relatively small molecules are conveyed, and
- the rate of drug transfer is low.

For example, US Patents 4,775,361, 5,165,418, and 5,423,803, and PCT Publication WO 97/07734 describe methods of using laser pulses to locally heat the stratum corneum to about 120°C, thereby causing local ablation, in order to cause a single hole to develop in the stratum corneum through which large molecules may pass. PCT Publication WO 97/07734 also discloses thermal ablation of the stratum corneum using an electrically-resistive element in contact with the stratum corneum, such that a high current through the element causes a general heating of tissue in its vicinity, most particularly the stratum corneum. Electroporation is well known in the art and is described, for example, in an article by Chizmadzhev et al., entitled, "Electrical properties of skin at moderate voltages, "*Biophysics Journal,* February, 1998, 74(2), pages 843-856, and in US Patent 5,019,034. All of these methods are characterized by at least one of the above-listed limitations.

US Patent 5,304,128 describes a syringe that includes a gas-driven piston to force liquid medication from the syringe through an injection nozzle. Pressure-driven devices of this sort obviate the need for a hypodermic needle, and they are therefore frequently called "needle-less injectors." Such devices must be able to produce and withstand very high gas pressure, which is needed to drive the medication through the stratum corneum. For this reason, such devices are costly and cannot practically be made as disposable products.

US Patents 5,860,957 and 5,983,131 describe apparatuses for injection of a substance through the stratum corneum of skin, comprising - besides from other elements: a reservoir containing the substance, the reservoir having at least one outlet; a pressure generator which applies a pressure to eject the substance through said at least one outlet; and one or more electrodes in a vicinity of the outlet which are placed on the skin, through which electrodes energy is conveyed to the skin to facilitate passage of the substance through the stratum corneum,

Furthermore, WO 95/15783 describes an apparatus for transdermal delivery of fluids in a non-invasive manner and without impediment of the skin's epidermis.

None of the above-mentioned publications teaches or suggests ablation of a portion of the stratum corneum responsive to the electric current conveyed into the skin in order to speed up of transdermal delivery of the substance. Accordingly, the working speed of their devices is relatively slow.

### SUMMARY OF THE INVENTION

It is the object of the present invention to provide an improved apparatus for increasing the speed of transdermal delivery of a substance. This is accomplished by the features of the characterizing part of claim 1.

Preferably - and unlike comparable drug-delivery systems known in the art - the substance is delivered to the patient in a painless manner, without the use of needles. The electrically-assisted injection device is preferably an inexpensive, single-use device, which requires no special skill to operate, and which administers the substance responsive to a single action by a nurse or the patient.

In some preferred embodiments of the present invention, parts of the device are constructed in a manner generally similar to a syringe, whereby a plunger, coupled to move within a barrel, generates the pressure in order to eject the substance. In these embodiments, the reservoir is preferably within the barrel, and the outlet comprises the nozzle of the syringe. Typically, the syringe is provided pre-filled with the substance, and the plunger is in a retracted position thereof. When the patient or the nurse applies a compressive force to the plunger, the electrodes are actuated, and the substance is ejected immediately thereafter. Alternatively, the syringe is a general-purpose drug-delivery device, which the nurse loads with a desired substance prior to administration.

For some applications, it is desirable to provide pressure in support or in place of that generated by manual compression of the plunger. This may be particularly useful when a relatively large quantity of the substance is to be delivered through the stratum corneum, as facilitated by the electrodes, to be passed deep into the dermis or into the bloodstream. In these cases, therefore, pressure is preferably generated by active methods, for example, one or more of the following:
- electrostatic force, generated within the barrel, which accelerates the substance to pass through the nozzle at high velocity,
- a small explosion, which creates a shock-wave in the barrel, to propel the substance at high velocity out of the syringe,
- a pre-tensed spring, which pulls or pushes the plunger, in order to drive the substance out of the barrel, and
- an electrolytic reaction, which rapidly increases the air-pressure in the barrel, driving out liquid substance.

Preferably, compression of the plunger actuates the electrodes to convey current to the patient's skin. In particular, movement of the plunger from the retracted position towards a compressed position thereof preferably closes an electric circuit, such that the current is enabled to flow from a charge-storage element, such as a capacitor or battery, into the patient's skin. Typically, the charge-storage element (and the syringe as a whole) is a single-use item, and is provided with sufficient charge stored therein to facilitate the flow of the substance, as provided by embodiments of the present invention. Alternatively, the syringe is designed for multiple administrations of one or more substances, and the charge-storage element is replaceable or rechargeable.

Preferably, use of a charge-storage element as described herein defines a maximum quantity of charge that may flow through the electrodes. Therefore, the element may be used to reduce or eliminate the possibility of undesired injury to the skin responsive to the passage of current therethrough. In some embodiments, the charge-storage element comprises resistors and other passive or active elements, which modify aspects of the current flow.

US Patent Application 09/189,170, filed 9 November 1998, entitled, "Transdermal drug delivery and analyte extraction," describes a device for enhancing transdermal movement of a substance. The device includes: (a) a skin patch, with at least two electrodes in contact with a subject's skin; and (b) a control unit, coupled to the patch, which causes a current to pass between the electrodes through the stratum corneum. Application of the current causes micro-channels to form in the stratum corneum to enable or augment transdermal movement of the substance. The control unit typically has switching circuitry to control the magnitude and/or duration of the electric field at the electrodes.

In some preferred embodiments of the present invention, the current flow generates micro-channels in the patient's skin, and thereby facilitates the desired passage of the substance through the skin. Micro-channel generation as practiced in these embodiments typically uses methods such as are described in the above-mentioned US Patent Application 09/189,170. The term "micro-channel," as used in the context of the present patent application, refers to a pathway generally extending from the surface of the skin through all or a significant part of the stratum corneum, through which pathway molecules can diffuse. Preferably, micro-channels allow the diffusion therethrough of large molecules at a greater rate than the same molecules would diffuse through pores generated by electroporation. The combination of such micro-channels with pressure-driven drug injection enables a far larger quantity of the medication to penetrate through the skin in a short time that would otherwise be possible.

Generally, the current flow between the electrodes can be described as having two components: (a) a perpendicular component, generally perpendicular to the skin surface; and (b) a lateral component, generally parallel to the skin surface. If the perpendicular component is too large, it may cause current to go through the stratum corneum into the underlying innervated, pain-sensitive, epidermal tissue and dermis.

Therefore, in embodiments of the present invention wherein micro-channels are generated, methods and/or apparatus are preferably employed to increase the relative value of the lateral component with respect to the perpendicular component. In general, the stratum corneum demonstrates a significantly higher resistance to the passage of molecules therethrough than does the underlying epidermal tissue. It is therefore an object of these embodiments to form micro-channels in the stratum corneum by ablating the stratum corneum, in order to increase conductance of the substance therethrough, generally without directly affecting or damaging epidermal tissue underlying the stratum corneum. Limiting current flow substantially to the non-innervated stratum corneum is believed to decrease or eliminate the patient's sensations, discomfort, or pain responsive to use of these embodiments of the present invention, particularly as compared with other injection procedures known in the art.

Alternatively or additionally, other electrically-mediated transdermal drug-delivery modalities known in the art are utilized to facilitate delivery of the substance, typically by decreasing resistance of the stratum corneum to the passage therethrough of the substance.

In some preferred embodiments of the present invention, an array of electrodes is deployed around the outlet, or around multiple outlets of the device, in order to further increase the transfer rate of the substance into the skin. Preferably, the array comprises closely-spaced electrodes, which generally act together to produce a high micro-channel density in an affected area of the skin. Alternatively or additionally, the array of electrodes conveys the current using other methods known in the art, in order to ablate or otherwise modify the stratum corneum, and thereby facilitate passage of the substance through the stratum corneum.

The present invention will be more fully understood from the following detailed description of the preferred embodiments thereof, taken together with the drawings, in which:

### BRIEF DESCRIPTION OF THE DRAWINGS

Figs. 1A and 1B are schematic, sectional illustrations of a device for electrically-mediated transdermal injection of a substance, in respective retracted and compressed positions thereof, in accordance with a preferred embodiment of the present invention;
Fig. 2 is a schematic, pictorial illustration of the device of Fig. 1A;
Fig. 3 is a schematic, pictorial illustration of another transdermal delivery device, in accordance with a preferred embodiment of the present invention; and
Figs. 4, 5, and 6 are schematic, sectional illustrations of still other transdermal injection devices, in accordance with preferred embodiments of the present invention.

### DETAILED DESCRIPTION OF PREFERRED EMBODIMENTS

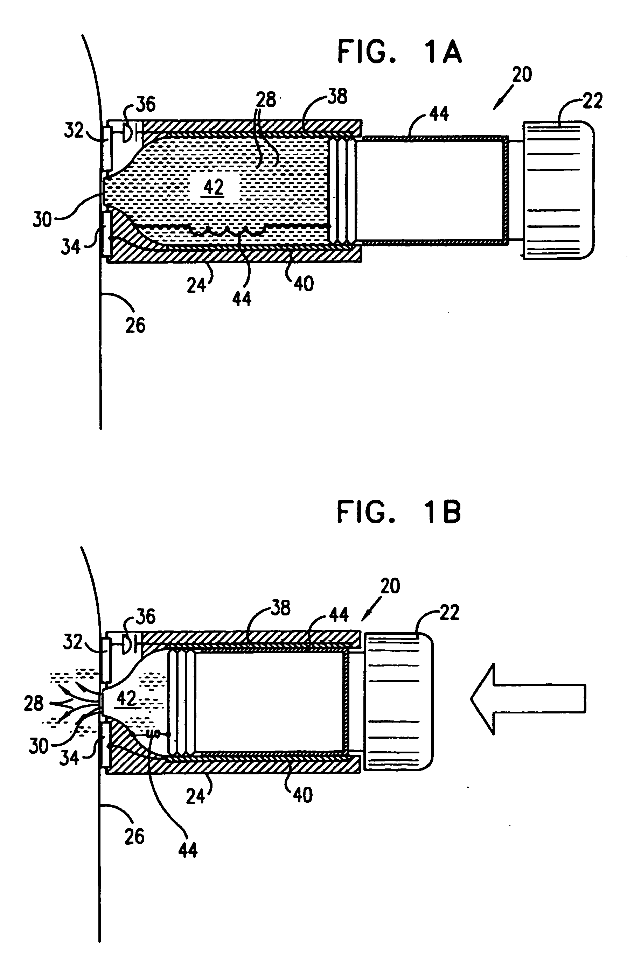

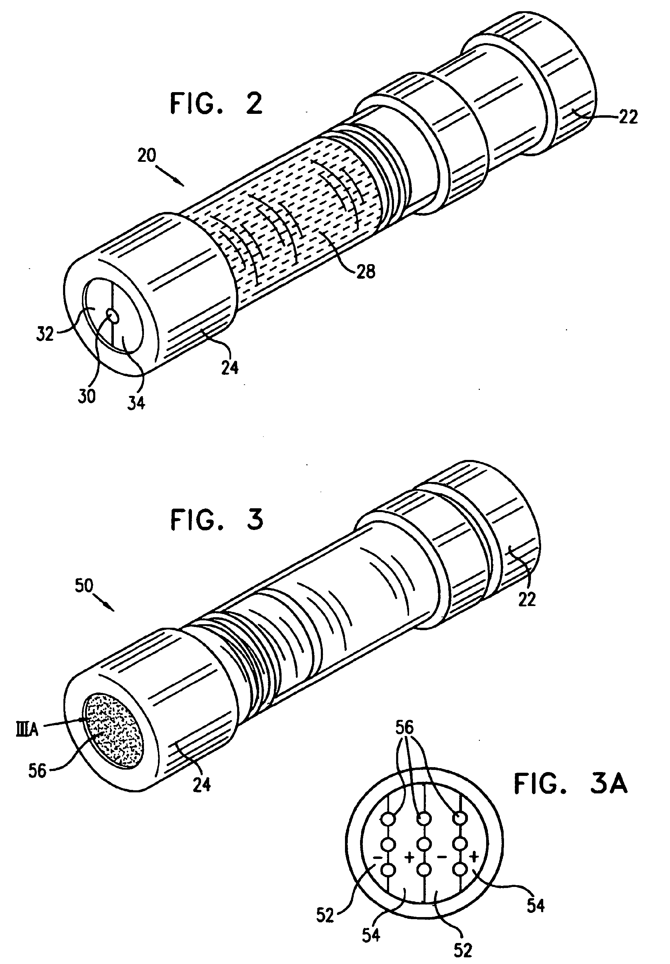

Reference is now made to Figs. 1A, 1B, and 2. Figs. 1A and 1B are schematic, sectional illustrations of a device 20 for delivery of a substance 28 into the skin 26 of a patient, in accordance with a preferred embodiment of the present invention. Fig. 2 is a schematic, pictorial illustration of device 20. Device 20 preferably has a form generally similar to that of a syringe, and is described herein with respect to two positions thereof: a retracted position, as shown in Fig. 1A, and a compressed position, as shown in Fig. 1B. In the retracted position, a plunger 22 is withdrawn from a barrel 24 of device 20. Movement of plunger 22 through the barrel into the compressed position preferably creates a pressure on a reservoir 42 containing substance 28, driving the substance out of the reservoir through an outlet 30, and into skin 26.

Preferably, device 20 comprises two electrodes 32 and 34, in a vicinity of outlet 30, which are placed on skin 26 prior to operating device 20. On actuation, electrical energy is conveyed through the electrodes to the skin. Application of electrical energy to the skin in this manner, as described further hereinbelow, facilitates passage of substance 28 through the stratum corneum of skin 26.

Preferably, compression of plunger 22 actuates the electrodes to convey current to the patient's skin. As shown in Figs. 1A and 1B, movement of the plunger from the retracted position towards the compressed position closes an electric circuit, by bringing a sliding, electrically-conductive member 44 into electrical contact with fixed electrically-conductive members 38 and 40, such that the current is enabled to flow from a charge-storage element 36 coupled to electrode 32 into the patient's skin.

Typically, charge-storage element 36 is designed for single-use, and comprises a capacitor (most preferably a super-capacitor, as is known in the art) or battery that is provided with sufficient charge stored therein to facilitate the delivery of the substance, as provided by embodiments of the present invention. Alternatively, device 20, including element 36, is designed for multiple administrations of one or more substances, and the charge-storage element is replaceable or rechargeable. For applications in which device 20 is required to have a long shelf-life, it is preferable to enable the device to be charged shortly before use, typically by exposing electrodes 32 and 34 and applying a voltage therebetween.

Because charge-storage element 36 is typically not connected to an external source of energy during operation of device 20, it generally can only release a quantity of charge through the electrodes that is less than or equal to the total charge stored in the element. For some applications, the total stored charge is regulated to reduce or eliminate the possibility of pain and/or undesired injury to the skin responsive to the passage of current therethrough.

In some embodiments, the charge-storage element comprises resistors and other passive or active elements (not shown in the figures), which modify aspects of the current flow to mitigate pain or injury. Although preferred embodiments of the present invention are described herein with respect to applying substantially constant current or voltage through electrodes 32 and 34, it is within the scope of the invention to apply more complex electric fields, for example, including alternating current components, square pulses, etc.

Preferably, the current flow from electrodes 32 and 34 generates micro-channels in skin 26, and thereby facilitates the desired passage of substance 28 through the skin. Micro-channel generation typically uses methods similar to those described in the above-cited US patent application, "Transdermal drug delivery and analyte extraction." In particular, apparatus and methods (e.g., stimulation parameters and electrode-placement parameters) described in that application are preferably used in some applications of the present invention to ablate a local region of the stratum corneum, in order to increase conductance of the substance therethrough, generally without damaging epidermal tissue underlying the stratum corneum. Limiting current flow substantially to the non-innervated stratum corneum is believed to decrease or eliminate the patient's sensations, discomfort, or pain responsive to use of these embodiments of the present invention.

Alternatively or additionally, one or more other electrically-mediated transdermal drug-delivery procedures known in the art, including, for example, electroporation or iontophoresis, are utilized to enhance the passage of substance 28 into the skin.

An optional mechanical-energy storage element 44, e.g., comprising a spring and/or a compressed-gas container, is coupled between plunger 22 and barrel 24. For applications in which element 44 comprises a spring, the spring is held in tension (or compression) when device 20 is in the retracted position. A locking mechanism (not shown) preferably prevents the spring from contracting, typically until plunger 22 has compressed a predetermined distance. The force which expels substance 28 from outlet 30 is preferably supplemented by the release of energy associated with the return of the spring to a neutral position thereof.

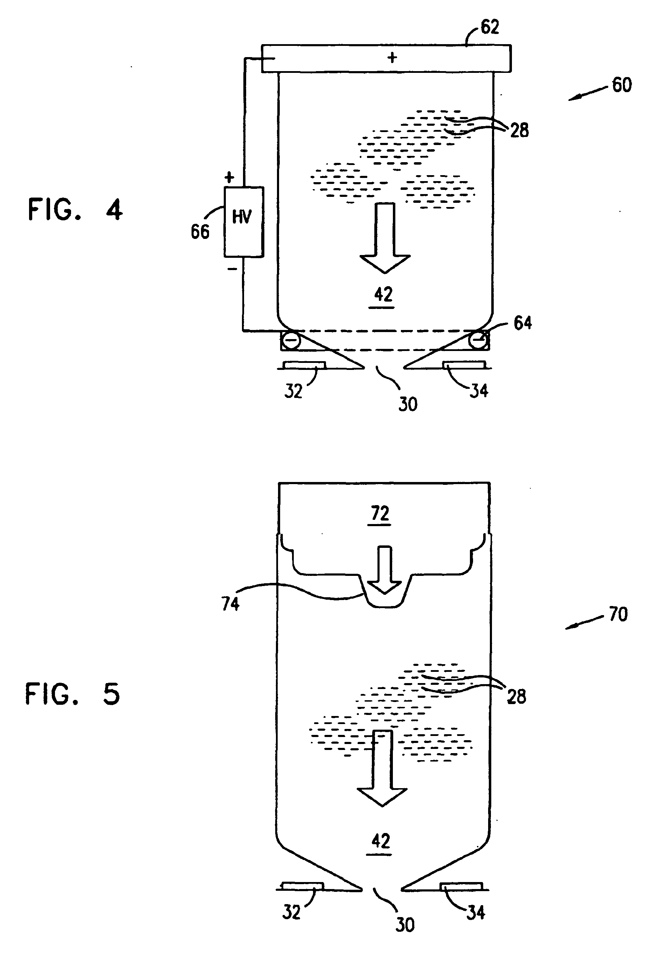

Typically, substance 28 comprises a therapeutic pharmaceutical product, such as a vaccine, or a diagnosis-related product, e.g., a radioactive compound. For most applications, substance 28 can be in a liquid or particulate form, although for particular embodiments of the present invention (for example, that described hereinbelow with reference to Fig. 4), one or the other form is preferred.

Fig. 3 is a schematic, pictorial illustration of another device 50 for transdermal delivery of substance 28, in accordance with a preferred embodiment of the present invention. Device 50 is similar in many respects to device 20, described hereinabove, differing generally only in the placement of electrodes and outlets. Preferably, device 50 comprises an array of negative and positive electrodes 52 and 54 disposed near a set of outlets 56 of reservoir 42. The electrode array may be linear, as shown in Fig. 3, or, for example, may comprise a grid of electrodes (not shown). When actuated, the electrodes generate an electric field, which ablates the stratum corneum or otherwise modifies properties of skin 26, in order to facilitate delivery of the substance into the patient's skin. Generally, placement of a plurality of positive and negative electrodes on skin 26, and/or the use of a plurality of reservoir-outlets, increases the rate of transfer of the substance into the patient's skin.

Reference is now made to Figs. 1A, 1B, 4, 5, and 6. For some applications, it is desirable to provide pressure in support or in place of that generated by manual compression of plunger 22. This may be particularly useful, for example, when a relatively large quantity of substance 28 is to be delivered through the stratum corneum, and then deep into the dermis, or into the bloodstream. In these cases, therefore, additional pressure is preferably generated by active methods, as described herein.

Fig. 4 is a schematic, sectional illustration of yet another device 60 for transdermal delivery of substance 28, in accordance with a preferred embodiment of the present invention. Device 60 is generally similar to device 20, described hereinabove with reference to Fig. 1A, but is distinguished therefrom by the use of a high voltage source 66, which is actuated by an operator of device 60 to apply a voltage between an upper surface 62 and a ring electrode 64, generally surrounding outlet 30. Preferably, substance 28 comprises powder particles, the particles in turn comprising an insulating, charge-carrying material.. Preferably, although not necessarily, the polarity between surface 62 and ring electrode 64 is alternated several times prior to administration of the substance in order to de-aggregate the powder particles. Thereafter, the electric field is generated between electrodes 32 and 34, in order to modify a property of the skin, e.g., to ablate the stratum corneum, and source 66 applies a voltage between electrodes 62 and 64, in order to accelerate the charged particles of substance 28 into the affected area of skin 26. The movement of substance 28 is optionally enhanced by manually compressing plunger 22 (not shown in this figure).

Some appropriate methods for generating the high voltage, for de-aggregating the powder, and for administering drugs in the powder form are described in US patent application 09/326,111, entitled, "Powder inhaler," which is assigned to the assignee of the present patent application and is incorporated herein by reference. In that application, a method is described for delivery of a dry powder to a patient by inhalation. The powder is de-aggregated and mobilized by application of a magnetic field. The field interacts with the package, engendering rapid motion thereof, which de-aggregates the powder.

Alternatively or additionally, useful techniques for applying electric fields to powders and managing drugs in the powder form, which may be utilized in some embodiments of the present invention, are described in US patent 5,983,135, entitled, "Transdermal delivery of fine powders," which is incorporated herein by reference. In that application, a powder delivery patch is described, including an electrostatic pad and an electrical power source. In preparation for application of a dry powder, such as a drug in powder form, to the skin of a subject, the power source applies an electrical potential to the pad, which causes the powder to adhere by electrostatic force to a lower side of the pad. This side is placed against the skin, and the electrical potential on the pad is reversed. The resultant electrostatic force drives the powder off the pad and onto the skin, through which the powder is absorbed into the body.

Fig. 5 is a schematic, sectional illustration of still another device 70 for transdermal delivery of substance 28, in accordance with a preferred embodiment of the present invention. Preferably, a small explosion is generated in a region 72 of device 70, such that a shock-wave 74 created by the explosion causes or enhances the flow of substance 28 through outlet 30 and into the region of skin 26 affected by electrodes 32 and 34. Alternatively or additionally, a non-explosive rapid expansion of gas is used to create a force to drive plunger 22 (not shown) to compress the substance and thus push it through the outlet. Further alternatively or additionally, the expanding gas directly compresses the substance or the reservoir, in order to expel the substance from device 70. Expanding gas can be generated inexpensively and safely by reacting, for example, citric acid and sodium bicarbonate. Still further alternatively or additionally, an electrolytic process is used to generate the gas, either to move the plunger or to directly drive the substance towards the outlet. In these examples of embodiments using expanding gas, some or all of the techniques and apparatus described hereinabove with reference to Figs. 1A, 1B, and 2-4 are optionally used to further enhance the desired transdermal movement of the substance.

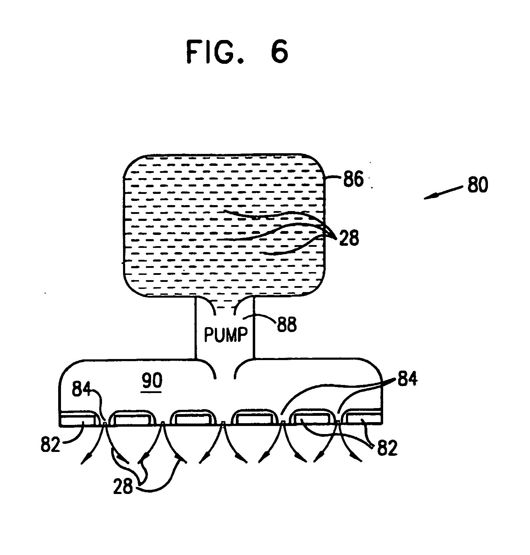

Fig. 6 is a schematic sectional illustration of an additional device 80 for transdermal delivery of substance 28, in accordance with a preferred embodiment of the present invention. Device 80 comprises a pump 88, preferably a two-chamber pump, which generates pressure to expel substance 28 from a reservoir 86 into an electrode compartment 90. On a lower surface of compartment 90, an array of electrodes 82 is preferably disposed in the proximity of a plurality of outlets 84. The substance preferably passes into skin 26 through the outlets, responsive to the pressure generated by pump 88 and to current injected into skin 26 by electrodes 82.

It will be appreciated that the individual preferred embodiments described above are cited by way of example, and that specific applications of the present invention will typically employ features described with reference to a plurality of the figures. The full scope of the invention is limited only by the claims.

## Claims

1. Apparatus (20) for injection of a substance (28) through the stratum corneum of skin (26), comprising:
a reservoir (42) containing the substance (28), the reservoir (42) having at least one outlet (30);
a pressure generator (22) which applies a pressure to eject the substance (28) through said at least one outlet (30); and
one or more electrodes (32, 34) in a vicinity of the outlet (30) which are placed on the skin (26), through which electrodes (32, 34) energy is conveyed to the skin (26) to facilitate passage of the substance (28) through the stratum corneum,
**characterized in that** the electrodes (32, 34) are suitable to convey electric current into the skin (26), such that a portion of the stratum corneum is ablated responsive to the current.

2. Apparatus according to claim 1, **characterized in that** the one or more electrodes (32, 34) comprise an array of two or more electrodes (32, 34) which convey current into the skin (26).

3. Apparatus according to claim 1, **characterized in that** the substance (28) comprises a powder having an electric charge associated therewith, and that the pressure generator (22) comprises first and second accelerating electrodes which generate an electric field to accelerate the powder and cause it to pass through said at least one outlet (30).

4. Apparatus according to claim 1, **characterized in that** it comprises a charge-storage element (36) which conveys electrical energy to the electrodes (32, 34).

5. Apparatus according to claim 1, **characterized in that** said at least one outlet (30) comprises a plurality of outlets from which the substance (28) is ejected.

6. Apparatus according to claim 1, wherein micro-channels are formed in the skin responsive to the current.

7. Apparatus according to any one of claims 1 to 5, **characterized in that** the pressure comprises pressure generated by an expanding gas.

8. Apparatus according to claim 7, **characterized in that** a shock-wave is generated responsive to the expansion of the gas, and that the substance (28) is ejected responsive to the shock-wave.

9. Apparatus according to claim 7, **characterized in that** an electrolytic reaction causes the gas to expand.

10. Apparatus according to any one of claims 1 to 5, **characterized in that** the reservoir (42) comprises a barrel (24), and that the pressure generator (22) comprises a plunger (22) slidably contained in the barrel (24), such that compression of the plunger (22) causes the substance (28) to be ejected from the outlet (30).

11. Apparatus according to claim 10, **characterized in that** the plunger (22) is compressed responsive to a manually-generated force and/or a gas-generated force and/or a spring-generated force.

12. Apparatus according to claim 10, **characterized in that** the electrodes (32, 34) apply electric current to the skin (26) responsive to compression of the plunger (22).

## Patentansprüche

1. Vorrichtung (20) zur Injektion einer Substanz (28) durch das *Stratum corneum* der Haut (26) hindurch, welche umfasst:
ein Reservoir (42), welches die Substanz (28) enthält und mindestens einen Auslass (30) aufweist;
einen Druckgenerator (22), welcher einen Druck erzeugt, um die Substanz (28) durch den erwähnten mindestens einen Auslass (30) auszustossen; und
eine oder mehrere in der Nachbarschaft des Auslasses (30) angeordnete Elektroden (32, 34) welche auf der Haut (26) platziert werden und durch welche Energie zur Haut (26) geschickt wird, um den Durchtritt der Substanz (28) durch das *Stratum corneum* hindurch zu erleichtern,
**dadurch gekennzeichnet, dass** die Elektroden (32, 34) geeignet sind, elektrischen Strom in die Haut (26) hinein leiten, so dass ein Teil des *Stratum corneum* als Reaktion auf den Strom abgetragen wird.

2. Vorrichtung nach Anspruch 1, **dadurch gekennzeichnet, dass** eine Anordnung von zwei oder mehr Elektroden (32, 34) vorgesehen ist, welche Strom in die Haut (26) hinein leiten.

3. Vorrichtung nach Anspruch 1, **dadurch gekennzeichnet, dass** die Substanz (28) ein elektrisch geladenes Pulver umfasst, und dass der Druckgenerator (22) erste und zweite Beschleunigungselektroden aufweist, welche ein elektrisches Feld erzeugen, um das Pulver zu beschleunigen und zu bewirken, dass dieses durch den genannten mindestens einen Auslass durchströmt (30).

4. Vorrichtung nach Anspruch 1, **dadurch gekennzeichnet, dass** sie ein Ladungsspeicher-Element (36) aufweist, welches den Elektroden (32, 34) elektrische Energie zuführt.

5. Vorrichtung nach Anspruch 1, **dadurch gekennzeichnet, dass** eine Mehrzahl von Auslässen (30) vorgesehen ist, durch welche die Substanz (28) ausgestossen wird.

6. Vorrichtung nach Anspruch 1, **dadurch gekennzeichnet, dass** in der Haut als Reaktion auf den Strom Mikrokanäle gebildet werden.

7. Vorrichtung nach einem der Ansprüche 1 bis 5, **dadurch gekennzeichnet, dass** der Druck durch ein expandierendes Gas erzeugten Druck umfasst.

8. Vorrichtung nach Anspruch 7, **dadurch gekennzeichnet, dass** als Reaktion auf die Expansion des Gases eine Schockwelle erzeugt wird, und dass die Substanz (28) als Reaktion auf die Schockwelle ausgestossen wird.

9. Vorrichtung nach Anspruch 7, **dadurch gekennzeichnet, dass** eine elektrolytische Reaktion die Expansion des Gases bewirkt.

10. Vorrichtung nach einem der Ansprüche 1 bis 5, **dadurch gekennzeichnet, dass** das Reservoir (42) ein Gehäuse (24) umfasst, und dass der Druckgenerator (22) einen im Gehäuse (24) verschiebbar angeordneten Kolben (22) aufweist, so dass das Pressen des Kolbens (22) bewirkt, dass die Substanz (28) durch den Auslass (30) ausgestossen wird.

11. Vorrichtung nach Anspruch 10, **dadurch gekennzeichnet, dass** der Kolben (22) als Reaktion auf eine manuell erzeugten Kraft und/oder eine durch Gas erzeugte Kraft und/oder eine Federkraft gepresst wird.

12. Vorrichtung nach Anspruch 10, **dadurch gekennzeichnet, dass** die Elektroden (32, 34) als Reaktion auf das Pressen des Kolbens (22) der Haut (26) elektrischen Strom zuführen.

## Revendications

1. Appareil (20) pour l'injection d'une substance (28) à travers la couche cornée de la peau (26), comprenant :
- un réservoir (42) contenant la substance (28), le réservoir (42) ayant au moins un orifice de sortie (30) ;
- un générateur de pression (22) qui applique une pression pour éjecter la substance (28) à travers le au moins un orifice de sortie (30) ; et
- une ou plusieurs électrodes (32, 34) à proximité de l'orifice de sortie (30) qui sont placées sur la peau (26), par l'intermédiaire desquelles électrodes (32, 34) de l'énergie est conduite vers la peau (26) pour faciliter le passage de la substance (28) à travers la couche cornée,
**caractérisé en ce que** les électrodes (32, 34) sont adaptées pour conduire le courant électrique jusqu'à la peau (26), de sorte qu'une partie de la couche cornée est ablatée du fait du courant.

2. Appareil selon la revendication 1, **caractérisé en ce que** la une ou plusieurs électrodes (32, 34) comprend une rangée de deux ou plus d'électrodes (32, 34) qui conduisent le courant jusqu'à la peau (26).

3. Appareil selon la revendication 1, **caractérisé en ce que** la substance (28) comprend une poudre ayant une charge électrique qui lui est associée, et **en ce que** le générateur de pression (22) comprend des première et seconde électrodes d'accélération qui génèrent un champ électrique pour accélérer la poudre et l'amener à passer à travers ledit au moins un orifice de sortie (30).

4. Appareil selon la revendication 1, **caractérisé en ce qu'**il comprend un élément de stockage de charge (36) qui conduit l'énergie électrique aux électrodes (32, 34).

5. Appareil selon la revendication 1, **caractérisé en ce que** ledit au moins un orifice de sortie (30) comprend une pluralité d'orifices de sortie à partir desquels la substance (28) est éjectée.

6. Appareil selon la revendication 1, dans lequel des micro canaux sont formés dans la peau du fait du courant.

7. Appareil selon l'une quelconque des revendications 1 à 5, **caractérisé en ce que** la pression comprend une pression générée par un gaz se dilatant.

8. Appareil selon la revendication 7, **caractérisé en ce qu'**une onde de choc est générée en réponse à la dilatation du gaz, et **en ce que** la substance (28) est éjectée en réponse à l'onde de choc.

9. Appareil selon la revendication 7, **caractérisé en ce qu'**une réaction électrolytique provoque l'expansion du gaz.

10. Appareil selon l'une quelconque des revendications 1 à 5, **caractérisé en ce que** le réservoir (42) comprend un cylindre (24), et **en ce que** le générateur de pression (22) comprend un piston (22) inclus à coulissement dans le cylindre (24), de sorte que la compression du piston (22) provoque l'éjection de la substance (28) par l'orifice de sortie (32, 34).

11. Appareil selon la revendication 10, **caractérisé en ce que** le piston (22) est comprimé en réponse à une force exercée manuellement et/ou une force générée par gaz et/ou une force générée par ressort.

12. Appareil selon la revendication 10, **caractérisé en ce que** les électrodes (32, 34) appliquent un courant électrique à la peau (26) en réponse à la compression du piston (22).
